# EUROPEAN PATENT APPLICATION

(11) **EP 3 056 240 A1**
(43) Date of publication of application: **17.08.2016**
(21) Application number: 16154007.5
(22) Date of filing: 03.02.2016
(51) Int. Cl.: A61M 25/01, A61M 25/00

(54) **DEVICE FOR IMPLANTATION OR INSERTION INTO THE HUMAN BODY WITH CHANGEABLE STIFFNESS**

(30) Priority: 13.02.2015 US 201562115656 P
(71) Applicant: BIOTRONIK SE & Co. KG, 12359 Berlin (DE)
(72) Inventor: Friedrich, Michael, 14532 Kleinmachnow (DE)
(74) Representative: Galander, Marcus

(57) **Abstract**

The application relates to a device (1) for implantation or insertion into the human body, and to a set with the device (1) and a stylet (12). In order to be able to change the flexibility of the device (1), the device (1) according to the invention comprises a pair (4) of stiffening elements (5, 6) that can be affixed to and detached from each other.

## Description

The invention relates to a device for implantation or insertion into the human body, with a tube, through which a cavity extends in the longitudinal direction of the tube. Furthermore, the invention relates to a set for implanting or inserting a device into the human body, with the device and a stylet that is adapted to be introduced into the human body.

Currently, devices, like catheters or leads for cardiac pacemakers or defibrillators, are introduced into the human body for purpose of examination, treatment or implantation. Such devices are flexible, in order to be able to be introduced into the human body without causing unnecessary damage. In case the device to be introduced into the human body is too flexible, stiffness modulators can be introduced into the device and in particular into its tube. Yet, with such a stiffness modulator, the stiffness of the complete device can be increased, only, hence, the increase acts along the entire length of the stiffness modulator. Furthermore, as the stiffness modulator needs to be introduced into the tube in order to change the stiffness, the stiffness cannot be changed in sections of the device which lay behind an element inside the tube in view of the stiffness modulator. For instance, a drug depot, a sensor and/or an electronic or electric component may be provided inside the tube, which hinders the stiffness modulator from being introduced farther into the tube.

Furthermore, shape modulators are known which may be introduced into the tube in order to shape the device. Yet, the same problems as mentioned above concerning the stiffness modulators appear when inserting the shape modulators.

In view of these disadvantages of the known devices, an object underlying the invention is to provide an implantable or insertable device and a set, wherein the stiffness and/or form of the device can be adapted in a more flexible manner.

The object is achieved according to the invention for the device mentioned in the beginning in that the device comprises at least one stiffening device with a pair of stiffening elements that are adapted to be affixed to each other in a stiffened state of the device, and to be detached from each other in a flexible state of the device. For the set for implanting or inserting, the object is achieved according to the invention in that the device is a device according to the invention.

As the pair of stiffening elements instead of the stiffness modulator determines the stiffness or flexibility of the device, the stiffness or flexibility is changeable in only a section of the device, namely the section in which the pair of stiffening elements is provided. The stiffness or flexibility of other sections of the device is not changed when the stiffness or flexibility of the section with the pair of stiffening elements is adapted. Hence, the stiffness of the section with the pair of stiffening elements can be adapted independent of the stiffness of other sections of the device, such that the stiffness of the device can be adapted in a more flexible manner.

The solutions according to the invention can be combined as desired and further improved by the further following embodiments that are advantageous on their own, in each case and unless stated to the contrary.

According to a first possible embodiment, the stiffening elements can be adapted to be affixed to each other with a form fit or a force fit. The form fit may be provided by a positive lock and the force fit may be provided by a friction lock, in particular a static friction lock. Thus, the design of the pair of stiffening elements can be chosen based on spatial or other requirements. The stiffening elements of the pair of stiffening elements are preferably arranged one after the other in the longitudinal direction. The stiffening element with the latch element and the other one of the stiffening elements with the counter-latch element may be affixed to an inner wall of the tube.

A simple form fit is provided in case one of the stiffening elements of the pair of stiffening elements is formed with a latch element, and the other one of the stiffening elements of the pair of stiffening elements is formed with a counter-latch element, wherein the stiffening elements are latched to each other in the stiffened state. Latch connections can be easily closed and opened, such that the stiffness of the device can be readily increased or decreased.

In a default state, the stiffening elements of the pair of stiffening elements can be latched or not latched with each other. Hence, by default, the device can have a high stiffness, which can be decreased by opening the latch connection, or a low stiffness, which can be increased by closing the latch connection of the stiffening elements.

The latch element may be formed with a latch protrusion and the counter-latch element may be formed with at least one latch recess for the latch protrusion. The latch recess may be essentially complementary to the latch protrusion, in order to provide a secure latch connection and in order not to require unnecessarily large installation space.

Due to the pair of stiffening elements, not only the stiffness may be set to a high or a low level. In particular, in case the stiffness is set to a high level, the stiffening elements may also define a form of the device and in particular of the tube, which may be a curved or a straight form. In order to be able to change the radius of a curved stiff state of the device, the counter-latch element may be formed with a plurality of latch recesses that are arranged after each other in the longitudinal direction. In case the latch protrusion engages the latch recess that is closest to the latch element in the longitudinal direction, the radius may be large. With an increased distance of the latch elements to each other, the smaller the radius of curvature of the device and in particular of the tube may be.

A device with only one pair of stiffening elements may provide sufficient stiffening of the device and in particular of the tube. Yet, in order to better define the plane in which the device and in particular the tube is stiffened, the device may comprise two pairs of stiffening elements that are arranged opposite of each other in the plane. Through the plane, a central axis of the tube may extend, in particular along the longitudinal direction.

In case the stiffness of the device shall be increasable in all directions perpendicular to the longitudinal direction, the device may comprise at least three pairs of stiffening elements that are preferably arranged in a plane that extends perpendicular to the longitudinal direction. In case the device comprises three pairs of stiffening elements, each of the pairs may be arranged in a corner of a triangle, preferably an equilateral triangle, which extends perpendicular to the longitudinal direction. In case four pairs of stiffening elements are provided, each of the pairs may be arranged in a different corner of a rectangle that extends perpendicular to the longitudinal direction. Hence, each of the pairs of stiffening elements may be provided in one corner of a polygon, whose amount of corners corresponds to the amount of pairs of stiffening elements, the polygon preferably being arranged perpendicular to the longitudinal direction and/or the central axis.

The latch element and/or the counter-latch element of the at least one pair of stiffening elements may extend into the cavity of the tube, such that the elements can be actuated by a stylet that is introducible into the cavity. Hence, the stiffness and the form of the device can be changed, even if the section whose stiffness or shape shall be changed is already introduced in the human body.

Preferably, the latch protrusion may have a rectangular cross section along the longitudinal direction. Hence, in case the latch element engages the complementary counter-latch elements, the latch connection cannot readily be opened by forces that act along the longitudinal direction. Yet, in order to be able to more easily open the latch connection or in order to be able to open the latch connection by a predefined force along the longitudinal direction, the latch element may have a tapering shape, for instance with a triangular cross section along the longitudinal direction. Even if the latch element has the triangular or V-shaped cross section, the latch connection can be secured by the stylet inserted in the cavity.

Latch and counter-latch elements that extend into the cavity require installation space, which may not be available and necessary for introducing other elements, for instance tools, into the tube or through the tube into the human body. Hence, in case installation space in the tube is limited, the stiffening elements are preferably adapted to be affixed to each other with a force fit.

For instance, one of the stiffening elements may be formed by the tube and the other one of the stiffening elements may be arranged in the cavity of the tube, wherein the stiffening elements are pressed against each other in the stiffened state. Preferably, the stiffening elements of the pair of stiffening elements are arranged one after the other perpendicular to the longitudinal direction. Thus, compared to the form fit, the pair of stiffening elements that are adapted to be affixed to each other by the force fit need a reduced amount of installation space, as at least one of the stiffening elements is provided by an element, namely the tube, which is already part of the device.

In a default state, the stiffening elements may not be pressed against each other, such that in the default state, the device has a low stiffness, i.e. a high flexibility, at least in the area of the pair of stiffening elements.

The other stiffening element may essentially be tube-shaped such that tools or other elements can be easily pushed through the other stiffening element. Furthermore, the other stiffening element may be more flexible radially than the one stiffening element, such that it can be easily deformed in the default state.

In case the stiffening elements are pressed against each other, for instance in case the other stiffening element is pressed against the one stiffening element from inside the other stiffening element in the radial direction of the tube, static friction between the stiffening elements prevents that the stiffening elements can be moved with respect to each other along the longitudinal direction. As changing the form of the device would result in a relative movement of the stiffening elements, the deformation of the device is at least impeded by the static friction, which needs to be overcome in order to deform the device.

In order to provide sufficient static friction, one of the stiffening elements may be formed of silicone, a metal, for instance a metal coil, or other suitable materials. The other one of the stiffening elements may comprise silicone or another suitable plastic, for instance polyurethane.

The flexibility of the other stiffening element can be increased by forming the other stiffening element with a radially expandable section. The increased flexibility of the radially expandable section compared to other sections of the other stiffening element may be provided by forming at least one slit into the radially expandable section. The at least one slit may extend along the longitudinal direction. The radially expandable section preferably comprises a plurality of slits, which are distributed along a circumferential direction of the tube shaped other stiffening element.

Furthermore, the radially expandable section may comprise at least one slit that extends under an angle to the longitudinal direction, the angle being between 0° and 90°, preferably between 40° and 70°, for instance 35°, 40° or 45°. The slits may be straight, curved or may even have a zigzag shape.

Furthermore, selected portions between the slits may be curved into the cavity, whereas portions adjacent to the curved portions are arranged at a distance in the radial direction of the other stiffening element to the selected portions. In particular, in case zigzagged slits are used, the selected portions increase the stiffness of the other stiffening element, in case the radial distance between the selected and the other portions is reduced such that the zigzag shapes of adjacent portions can mesh with each other. In order to bring the selected portions closer to the other portions in the radial direction, the stylet can be inserted into the cavity and in particular into the other stiffening element.

The slits may furthermore be shaped spirally. The spirally shaped slits may extend in a selected circumferential direction of the other stiffening element. In case one of the stiffening elements is rotated against the circumferential direction and with respect to the other end of the stiffening element, portions between the slit are deformed, such that the radially expandable section seeks to move in the radial direction and presses against the one stiffening element, thereby providing the static friction.

Furthermore, independent of the above, a device with a tube that comprises a spirally shaped slit is advantageous, as a torque directed in the circumferential direction in which the spirally shaped slit extends can be transmitted via such a tube, whereas torques in the opposite direction are not transmitted or transmitted in a reduced manner. For instance, a helical fixation screw at the tip of a pacemaker or defibrillator lead may be retracted from the heart tissue unhindered by turning in one circumferential direction, whereas when screwing in the opposite circumferential direction the transmitted torque is limited, thus preventing any damage to tissue. As an alternative to the other stiffening element formed with slits, a wire coil, for instance of a round wire, may be provided. The form fit may be provided without arranging latch elements inside of the tube.

Furthermore, the pair of stiffening elements may be provided by a stiffening device, which may be essentially tube-shaped, such that the stiffening device can be introduced into the tube. Alternatively, the tube can be introduced into the stiffening device. The stiffening device may comprise two end rings, which are interconnected by the stiffening elements, which may be stiffening bars. The stiffening bars may comprise a zigzag shape or may be toothed rods. When rotating one end ring relative to the other end ring in a circumferential direction of the stiffening device, adjacent stiffening bars are brought in contact with each other, such that the zigzag shaped or toothed rods engage with each other, thereby blocking a further movement of the one end ring with respect to the other end ring. Furthermore, due to the positive fit of the stiffening bars, the stiffness of the stiffening device increases not only when a torque is applied to the stiffening device but also in case forces perpendicular to the longitudinal direction act on the stiffening device such that stiffness is increased.

In order to bring the stiffening bars into engagement without applying torque forces onto the end rings, displacement elements, e.g. displacement balls or displacement bars, can be introduced between the stiffening bars, for instance, when the stylet is introduced into the device. The stylet forces the displacement elements to move in the radial direction, thereby deforming the stiffening bars in the radial direction until they engage with the respective adjacent stiffening bar. The displacement bars may be sections of round wire.

The invention is described hereinafter in greater detail and in exemplary manner using advantageous embodiments and with reference to the drawings. The described embodiments are only possible configurations, in which, however, the individual features as described above can be provided independently of one another or can be omitted in the drawings.
- Figs. 1 to 6: are schematic cross-sectional views of exemplary embodiments of pairs of stiffening elements adapted to be affixed with a form fit,
- Figs. 7 and 8: is a schematic perspective and partly sectional view of another exemplary embodiment of the device according to the invention,
- Figs. 9 to 15: are schematic views of embodiments of stiffening elements of the device according to the invention,
- Figs. 16 and 17: show is schematic perspective views of another exemplary embodiment of a stiffening device according to the invention,
- Figs. 18 and 19: show schematic cross-sectional views of another exemplary embodiment of the device, and
- Figs. 20 to 23: are schematic side views of exemplary embodiments of stylets of the set according to the invention.

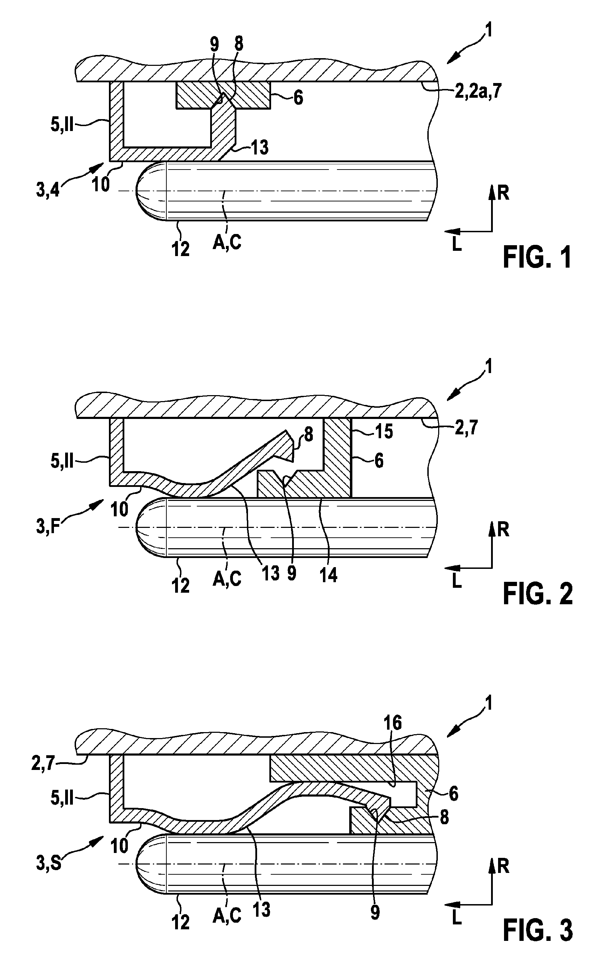

First, a device 1 according to the invention is described with respect to the exemplary embodiment of Figure 1. The device 1 is only partly shown in a cross-sectional view along a longitudinal direction L. The device 1 comprises a tube 2, of which only one side wall 2a is shown in the cross-sectional view of Figure 1. The device 1 further comprises a stiffening device 3, with which the stiffness of the device 1 and in particular of its tube 2 can be changed. For instance, the stiffening device 3 comprises a pair 4 of stiffening elements 5, 6. The stiffening elements 5, 6 are each affixed to the tube 2, for instance at an inner wall 7 of the side wall 2a. The stiffening elements 5, 6 are adapted to be affixed to each other, in order to provide a device 1, e.g. a tube 2, with a high stiffness. Such a stiffened state S is shown in the exemplary embodiment of Figure 1. Hence, the exemplary embodiment of Figure 1 provides a device 1 with a normally flexible state. I.e. the stiffness of the device 1 can be increased by affixing the stiffening elements 5, 6 to each other.

In case the stiffening elements 5, 6 are not directly affixed to each other, the device 1 and in particular its tube 2 is in its flexible state, which is, however, not shown in the exemplary embodiment of Figure 1.

The stiffening elements 5, 6 of the exemplary embodiment of Figure 1 are adapted to be affixed to each other with a form fit, in particular by a latch connection. The stiffening elements 5, 6 may comprise a latch element 8, and stiffening element 6 may be provided with a counter-latch element 9 for the latch element 8. Preferably, the counter-latch element 9 is at least sectionwise formed complementary to the latch element 8.

The latch element 8 may be formed as a latch protrusion and the counter-latch element 9 may be formed as a latch recess, which the latch protrusion engages in the stiffened state S. The counter-latch element 9 opens away from the inner wall 7 and against a radial direction R of the tube 2, the radial direction R extending perpendicular to the longitudinal direction L.

For instance, stiffening element 5 is formed with a latch arm, to which latch element 8 is provided. Latch arm 10 can be swiveled towards the counter-latch element 9 such that latch element 8 is introduced into the counter-latch element 9 until it contacts the stiffening element 6. The latch arm 10 preferably extends essentially parallel to the longitudinal direction L and may in particular extend in or against the longitudinal direction L as seen from the latch element.

The latch arm 10 may be affixed to a holding section 11 of the stiffening element 5, the holding section 11 being affixed to the tube 2 and in particular to its inner wall 7. The holding section 11 may be a holding bar that extends in a direction, whose components at least partly point parallel to the radial direction R.

In Figure 1, the stiffening device 3 is shown in the stiffened state S, in which the latch element 11 is pressed in the radial direction R by a stylet 12 that is inserted into the tube 2 along the longitudinal direction L. A central axis A of the stylet 12 may extend parallel to or even overlap a central axis C of the tube 2, in case the stylet 12 is introduced in the tube 2.

In order to deflect the latch element 8 in the radial direction R by the stylet 12, the stiffening element 5 may comprise a deflection surface 13, which is slanted with respect to the longitudinal direction L, such that a normal vector of the deflection surface 13 extends against the longitudinal direction L and the radial direction R. As soon as the stylet 12 contacts the deflection surface 13 and the stylet 12 is further moved in the longitudinal direction L, the latch element 8 is deflected in the radial direction R. The deflection surface 13 of the exemplary embodiment of Figure 1 is for instance arranged at a free end of the latch arm 10, the free end pointing against the longitudinal direction L. In order to able to safely actuate the stiffening device 3 and in particular to move the latch element 8 in the radial direction R, the deflection surface 13 is preferably arranged on the same side of the central axis C as the stiffening element 6.

In case stylet 12 is not inserted in the tube 2 and does not contact the stiffening element 5, the latch element 8 is in a default unlatched position, which is before the shown latched position in the radial direction R.

Figure 2 shows another exemplary embodiment of the device 1 according to the invention. For elements which correspond in form and/or function to elements of the exemplary embodiment of Figure 1, same reference numerals are used. For the sake of brevity, only differences to the exemplary embodiment of Figure 1 are referred to.

The stiffening elements 5, 6 of the pair 4 of stiffening elements 5, 6 of the stiffening device 3 differ from the exemplary embodiments of Figure 1. In contrast to the exemplary embodiment of Figure 1, the exemplary embodiment of Figure 2 provides a device 1 with a normally stiff state, hence, the stiffness of the device 1 can be decreased by actuating the stiffening device 3.

The device 1 of Figure 2 is shown in its flexible state F, in which the stiffening element 5 is actuated and is not affixed to the other stiffening element 6. In particular, latch arm 10 is deflected in the radial direction R towards the side wall 7 of the tube 2, such that latch element 8 does not engage counter-latch element 9, thereby affixing the stiffening elements 5, 6 to each other.

In the default position of the latch element 8, latch element 8 engages the counter-latch element 9. In the flexible state F, latch element 8 is moved away from the counter-latch element 9 in the radial direction R.

Stiffening element 6 comprises the counter-latch element 9, which opens in the radial direction R and in particular away from the central axis C towards the side wall 7 of the tube 2. In order to be able to arrange the latch element 8 between the counter-latch element 9 and the closest section of the inner wall 7, counter-latch element 9 is formed in a latch section 14 of the stiffening element 6, the latch section 14 being arranged at a distance to the closest inner wall 7. In order to interconnect the inner wall 7 and the latch section 14, stiffening element 6 comprises a holding section 15, which may be designated as holding bar or arm, which extends from the inner wall 7 towards the central axis C, for instance against the radial direction R.

The latch section 14 and the holding section 15 may contact each other at an end of the latch section 14 that points in or against the longitudinal direction L. The latch section 14 may protrude from the holding section 15 in or against the longitudinal direction L. Hence, the stiffening element 6 of the exemplary embodiment of Figure 2 opens in or against the longitudinal direction L, such that at least the latch element 8 of the stiffening element 5 can project into a free space between the counter-latch element 9 and the closest inner wall 7.

Latch arm 10 may be provided with the deflection surface 13, which, however, is preferably arranged between the latch element 8 and the holding section 11 of the stiffening element 5. In the default position of the latch arm 10, the deflection surface 13 is closer to the central axis C than the latch section 14. Hence, when introducing the stylet 12 into the longitudinal direction L into the tube 2, the stylet 12 can pass the stiffening element 5 until it abuts the deflection surface 13. When further introducing the stylet 12 into the tube 2, the interaction between the stylet 12 and the deflection surface 13 forces the latch arm 10 away from the central axis C and towards the closest inner wall 7, such that latch element 8 is brought out of its default latched connection with the counter-latch element 9.

In order to provide the deflection surface 13, latch arm 10 can be curved and may arch away from the inner wall 7, which it contacts. Alternatively, latch arm 10 may be provided with a deflection projection, which protrudes from the latch arm 10 towards the central axis C, the deflection projection comprising the deflection surface 13. In order to able to safely actuate the stiffening device 3 and in particular to move the latch element 8 in the radial direction R, the deflection surface 13 is preferably arranged on the same side of the central axis C as the stiffening element 6.

Figure 3 shows another exemplary embodiment of the device 1 according to the invention. Same reference signs are being used for elements, which correspond in function and/or structure to the elements of the previous exemplary embodiments. For the sake of brevity, only the differences from the previous exemplary embodiments are looked at.

The stiffening device 3 of the exemplary embodiment of Figure 3 has a normally flexible state. The stiffening device 3, however, is shown in its stiffened state S, in which the stiffening elements 5, 6 are affixed to each other.

In addition to the stiffening element 6 of the exemplary embodiment of Figure 2, the stiffening element 6 of Figure 3 may comprise an optional abutment section 16, which extends parallel to the longitudinal direction L and which provides for an abutment for the stiffening element 5 and in particular for its latch arm 10. The abutment section 10 preferably lies flat against the inner wall 7.

In the flexible state F of the stiffening device 3, latch element 8 is preferably closer to the inner wall 7 and/or the abutment section 10, than in the shown stiffened state S. In case latch arm 10 is deflected towards the inner wall 7 and away from the central axis C, for instance by the stylet 12, latch arm 10 contacts the optional abutment section 16 and is bent by the abutment section 16 such that latch element 8 is moved against the radial direction R and towards the counter-latch element 9, in case latch arm 10 is further deformed towards the inner wall 7. Alternatively to the abutment section 16, the latch arm 10 may contact the inner wall 7 and may be bent by the tube 2. However, in case the tube 2 would be mechanically overloaded, the abutment section 16 may be provided.

In the longitudinal direction L, the latch arm 10 may be curved away from the inner wall 7 and towards the central axis C between the holding arm 11 and the deflection surface 13. In the longitudinal direction L before the deflection surface 13, the latch arm 10 is preferably curved in an opposite direction, in particular away from the inner wall 7 and towards the central axis C. Hence, the latch arm 10 may have a form essentially corresponding to the letter S.

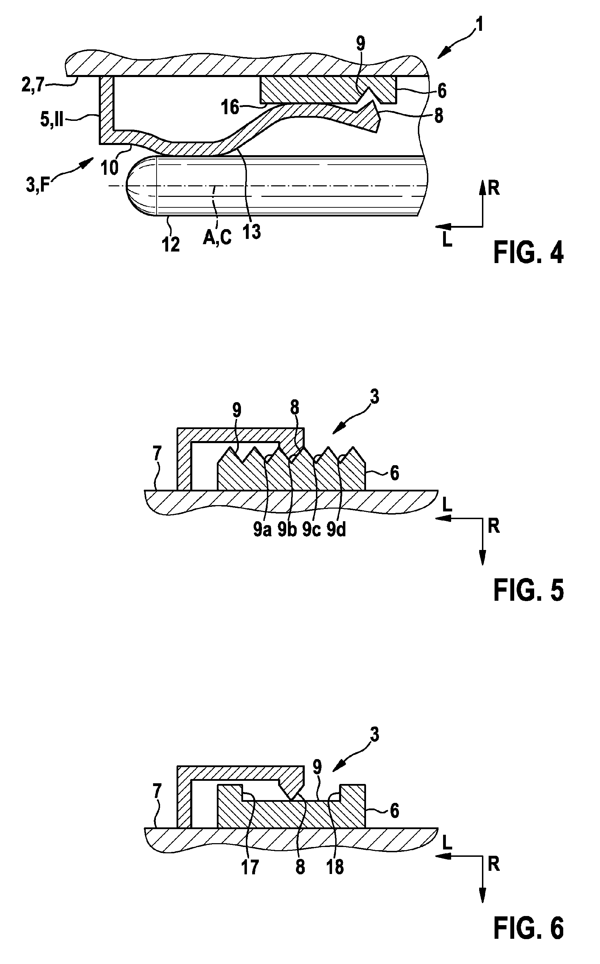

Figure 4 shows another exemplary embodiment of the device 1 according to the invention. Same reference signs are being used for elements, which correspond in function and/or structure to the elements of the previous exemplary embodiments. For the sake of brevity, only the differences from the exemplary embodiment of Figure 3 are looked at.

The stiffening element 5 of the exemplary embodiment of Figure 4 essentially corresponds to the stiffening element 5 of the exemplary embodiment of Figure 3. However, latch element 8 of the stiffening element 5 according to the exemplary embodiment of Figure 4 faces in an opposite direction compared to the latch element 8 of the exemplary embodiment shown in Figure 3 and in particular towards the inner wall 7 and away from the central axis C.

Furthermore, stiffening element 6 essentially corresponds to stiffening element 6 of the exemplary embodiment of Figure 1. In addition, stiffening element 6 of the exemplary embodiment of Figure 4 comprises the abutment section 16, which is arranged after the counter-latch element 9 in or against the longitudinal direction L.

Due to the different arrangement of the latch element 8 and the abutment section 16, the stiffening device 3 of the exemplary embodiment of Figure 4 has a normally stiffened state. Figure 4, again, shows the stiffening device 3 in the actuated state that is the flexible state F in the exemplary embodiment of Figure 4, I which the latch element 8 is forced away from the counter latch element 9 by introduction of a movement into the deflection surface 13 away from the central axis C, this movement being introduced into the latch element 8 in a redirected manner by interaction between the abutment section 16 and the latch arm 10.

Figure 5 shows another exemplary embodiment of the stiffening device 3 according to the invention. Same reference signs are being used for elements, which correspond in function and/or structure to the elements of the previous exemplary embodiments of Figures 1 to 4. For the sake of brevity, only the differences from the previous exemplary embodiments are looked at.

The stiffening element 6 comprises not only one, but a plurality and in particular five counter-latch elements 9, 9a, 9b, 9c, 9d. The counter-latch elements 9, 9a to d are arranged one after the other in the longitudinal direction L such that the latch element 8 can engage in any of the counter-latch elements 9, 9a to d in different states of the tube 2. Hence, tube 2 can be stiffened not only in one predetermined form, but in different predetermined forms, the number of the predetermined forms corresponding to the number of counter-latch elements 9, 9a to 9d. The different predetermined forms may have different radii of curvature.

All of the stiffening elements 5, 6 can be provided with a plurality of counter-latch elements 9 in order to provide that the tube 2 can be stiffened in a plurality of curved forms.

Figure 6 shows another embodiment of the stiffening device 3 in a schematic cross-sectional view. Same reference signs are being used for elements, which correspond in function and/or structure to the elements of the previous exemplary embodiments. For the sake of brevity, only the differences from the exemplary embodiment of Figures 1 to 5 are looked at.

In the longitudinal direction L the counter-latch element 9, which is formed as a latch recess, is larger than the latch element 8, which is formed as a latch protrusion. Hence, in case device 1 comprises the stiffening device 3 according to the exemplary embodiment of Figure 6, the tube 2 can be flexibly deformed in a certain range, whereas stiffness of the tube 2 increases as soon as latch element 8 contacts stop protrusions 17, 18 of the stiffening element 6, the stop protrusions 17, 18 protruding from the inner wall 7 towards the central axis C. In the longitudinal direction L, the latch recess is arranged between the stop protrusions 17, 18.

All of the stiffening elements 5, 6 can be provided with one counter-latch element 9 or a plurality of counter-latch elements 9. The plurality of counter-latch elements 9 are preferably arranged one after the other in the longitudinal direction L.

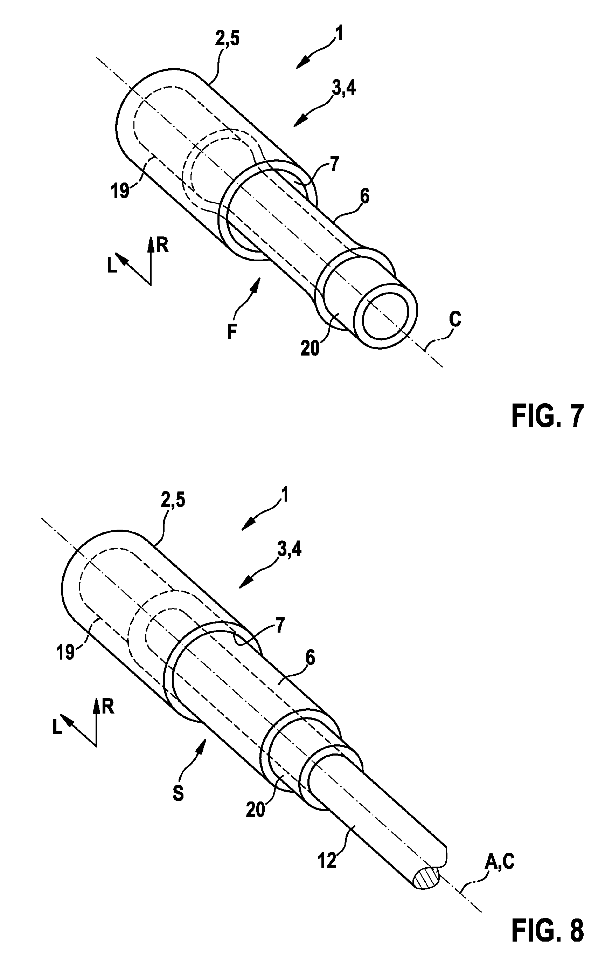

Figure 7 shows another exemplary embodiment of the device 1 in a schematic perspective view. Same reference signs are being used for elements, which correspond in function and/or structure to the elements of the previous exemplary embodiments. For the sake of brevity, only the differences from the exemplary embodiment of Figures 1 to 6 are looked at.

The device 1 of Figure 7 is shown as a partly exposed view and with another exemplary embodiment of the stiffening device 3, wherein the stiffening elements 5, 6 of the pair 4 of such a stiffening device 3 are adapted to be affixed to each other with a force fit, in particular by static friction between the stiffening elements 5, 6.

The stiffening element 5 may be provided by the tube 2. The other stiffening element 6 is preferably arranged inside of the tube 2 and may have a tube shape.

In the exemplary embodiment of Figure 7, the stiffening element 6 is at least sectionwise or even completely arranged at a distance from the inner wall 7 of the tube 2, such that the stiffening elements 5, 6 do not extensively contact each other. In particular, the other stiffening element 6 is shown in its default state, in which it is contracted towards the central axis C, for instance due to elastic forces inherent to a radially elastic section 6q of the other stiffening element 6. The device 1 of Figure 7, hence, has a default flexible state F. In the flexible state F, an inner diameter of the tube-shaped stiffening element 6 and in particular of its radially flexible section 6a is less than an inner diameter of at least one of the positioning elements 19, 20.

In the longitudinal direction L, the other stiffening element 6 may be arranged between positioning elements 19, 20, the positioning elements 19, 20 for instance defining the position of the other stiffening element 6 along the longitudinal direction L within the tube 2. The positioning elements 19, 20 are preferably tube shaped, such that for instance stylet 12 can be guided through one of the positioning elements 19, 20, in particular to the other stiffening element 6. The other stiffening element 6 may be affixed to the positioning elements 19, 20, for instance by a material fit, like gluing, or by a force fit, for instance by static friction. Other possibilities for affixing the other stiffening element 6 to the positioning elements 19, 20 are welding or stitching.

Figure 8 shows the exemplary embodiment of Figure 7, wherein the device 1 of Figure 8 is shown in its stiffened state S.

The stylet 12 is introduced into the device 1 and in particular into its tube 2 and/or into at least one of the positioning elements 19, 20, such that stylet 12 is at least sectionwise arranged within the other stiffening element 6.

Preferably, the stylet 12 has an outer diameter perpendicular to its central axis A, that essentially corresponds to the inner diameter of at least one of the positioning elements 19, 20. In case the stylet 12 is introduced into the other stiffening element 6, the radially flexible section 6a of the tube-shaped stiffening element 6 is forced away from the central axis C and towards the inner wall 7, against which it is pressed.

In a state, in which the stiffening elements 5, 6 are pressed against each other, static friction between the stiffening elements 5, 6 causes that a relative movement between the tube 2 and the other stiffening element 6 is at least hindered. As bending of the device 1 in the area of the other stiffening element 6 would cause such a relative movement, the static friction increases the stiffness of the device 1 at least in the area of the other stiffening element 6.

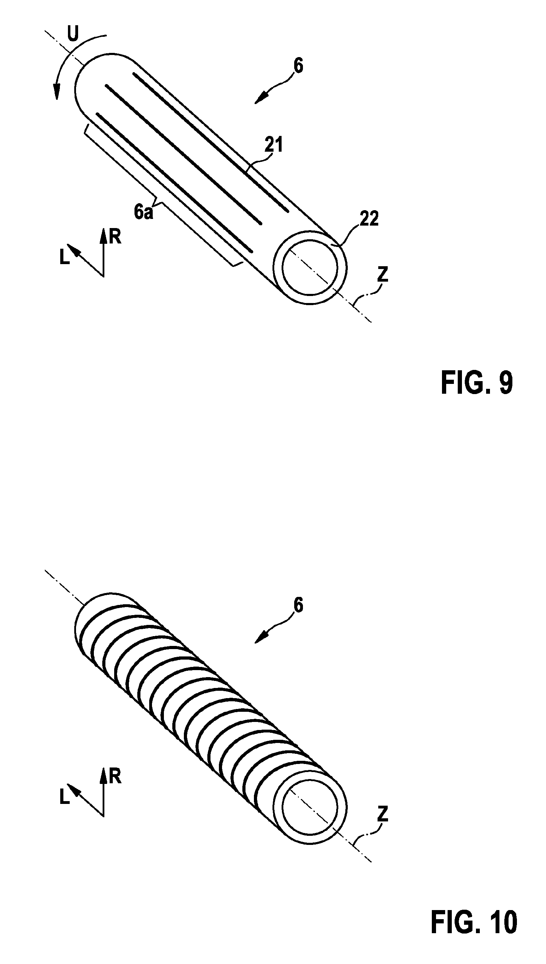

Figures 9 to 15 show different embodiments of the tube-shaped other stiffening element 6.

The other stiffening element 6 of the exemplary embodiment of Figure 9 comprises a plurality of slits 21 that extend along the longitudinal direction L. The slits 21 are distributed along a circumferential direction U of the tube-shaped other stiffening element 6. Preferably, the slits completely extend through a side wall 22 of the tube-shaped stiffening element 6, in particular along the radial direction and away from a central axis Z of the tube-shaped stiffening element6, and end before longitudinal ends of the tube shaped stiffening element 6.

The tube-shaped other stiffening element 6 of the exemplary embodiment of Figure 10 is formed of a wire, which is arranged around the central axis Z to form a coil.

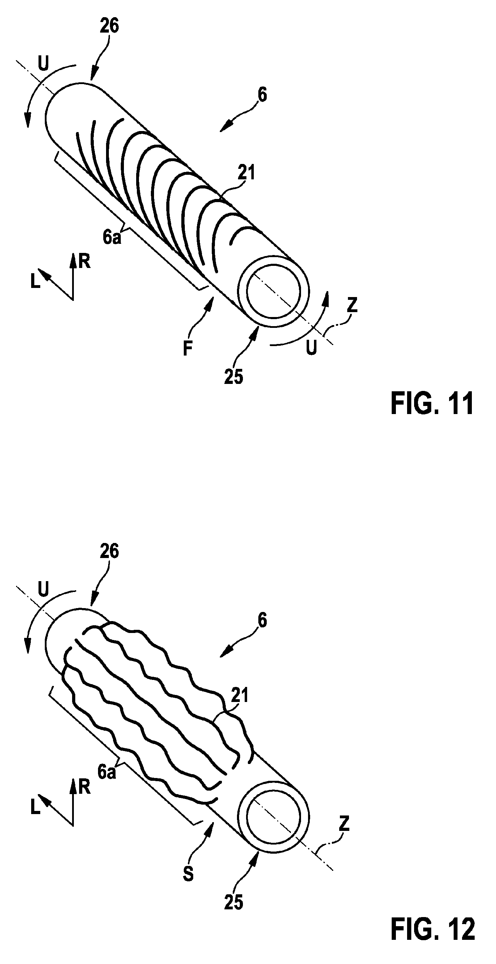

Figure 11 shows another exemplary embodiment of the other stiffening element 6 with a spirally formed slit 21 in its radially flexible section 6a. The spiral slit 21 extends in the circumferential direction U and against the longitudinal direction L. When rotating one distal end 25 in the circumferential direction U with respect to the opposite distal end 26 of the other stiffening element 6, a torque in the circumferential direction U can be transmitted. As will become obvious from the following description of Figure 12, Figure 11 shows the other stiffening element 6 in a flexible state of the device 1.

Figure 12 shows the other stiffening element 6 of the exemplary embodiment of Figure 11 in a stiffened state S of the device 1. Distal end 25 has been rotated against the circumferential direction with respect to the other distal end 26, such that the spirally shaped radially flexible section 6a has been deformed to a shape with essentially longitudinal slits 21 and, thus, expanded in the radial direction and away from the central axis Z. Hence, at least the radially flexible section 6a is pressed against the tube 2 and creates static friction with the inner wall 7.

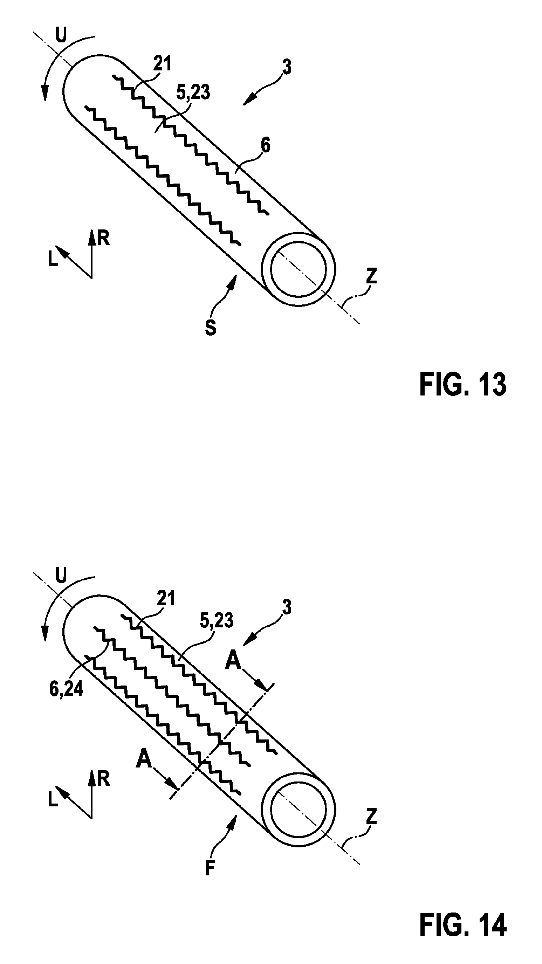

Figure 13 shows another exemplary embodiment of the stiffening device 3 with slits 21 that essentially extend along the longitudinal direction L of the tube shaped stiffening device 3, the slits 21 being zigzag shaped.

In the circumferential direction U, stiffening elements 5, 6 formed as stiffening bars 23 are separated from each other by two of the slits 21, the two slits 21 being two consecutive slits 21 in the circumferential direction U. Teeth formed by the zigzag shaped slits 21 may be designated as latch protrusions that may engage each other.

In the default state of the stiffening device 3 shown in Figure 13, adjacent stiffening bars 23 engage with each other, such that deformation around the radial direction R at least in the area of the slits 21 is hindered, such that the stiffening device 3 is in its stiffened state S. In order to change the stiffness of the stiffening device 3 adjacent stiffening bars 23 have to be brought out of engagement with each other.

Figure 14 shows another exemplary embodiment of the stiffening device 3. Same reference signs are being used for elements, which correspond in function and/or structure to the elements of the exemplary embodiment of Figure 13. For the sake of brevity, only the differences from the exemplary embodiment of Figure 13 are looked at.

The stiffening device 3 of the exemplary embodiment of Figure 14 comprises the zigzaged slits 21 and the stiffening bars 23. Furthermore, between two of the stiffening bars 23 in the circumferential direction U, another stiffening bar 24 is arranged. Hence, teeth formed by the zigzag shaped slits 21 may be designated as latch protrusions that may engage each other. However, in the default state of the stiffening device 3, the stiffening bars 24 are at least sectionwise out of engagement with the stiffening bars 23. For instance, the stiffening bars 24 may extend towards the central axis Z, whereas the stiffening bars 23 essentially extend along the longitudinal direction L with a constant distance to the central axis Z. The stiffening bars 23 may be designated as the stiffening elements 5 and the stiffening bars 24 may be designated as the other stiffening elements 6.

As consecutive stiffening bars 23, 24 are not in engagement with each other in a default state of the stiffening device 3, the stiffening device 3 of the exemplary embodiment of Figure 14 has a default flexible state F.

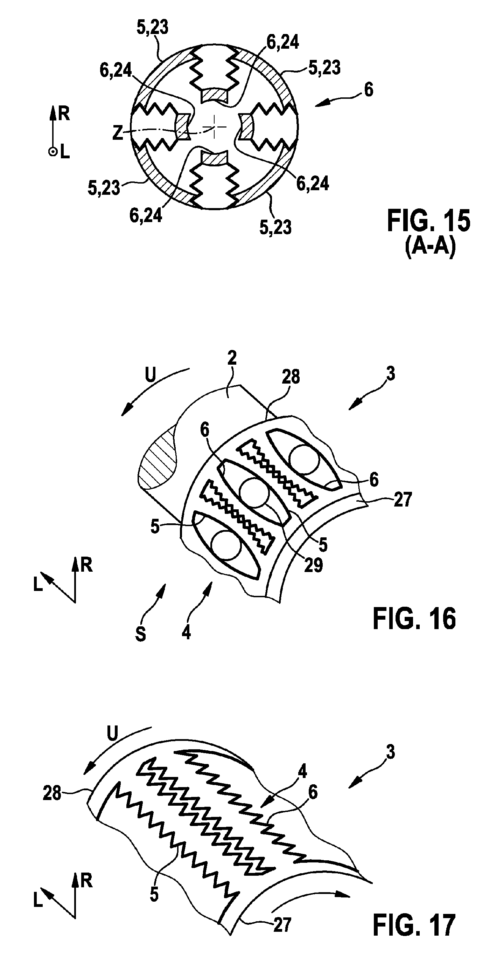

Figure 15 shows the stiffening device 3 according to the exemplary embodiment of Figure 14 in a cross-sectional view perpendicular to the central axis Z. It is clearly shown that the stiffening bars 24 extend towards the central axis Z whereas stiffening bars 23 have an essentially straight shape parallel to the central axis Z. When introducing the stylet 12 into the stiffening device 3, the curved stiffening bars 24 are forced away from the central axis Z, until they are in engagement with the straight stiffening bars 23. Hence, by introducing the stylet 12 into the stiffening device 3, the stiffening device 3 of the exemplary embodiment of Figures 14 and 15 can be transformed from a flexible to a stiffened stiffening device 3.

Figure 16 partly shows an exemplary embodiment of the stiffening device 3 in a perspective view. Same reference signs are being used for elements, which correspond in function and/or structure to the elements of the previous exemplary embodiments. For the sake of brevity, only the differences from the exemplary embodiments of the previous figures are looked at.

The stiffening device 3 is shown arranged on the tube 2. Alternatively, the stiffening device 3 may be arranged inside of the tube 2. The stiffening device 3 is essentially tube-shaped and comprises two end rings 27, 28, that are arranged one after the other in the longitudinal direction L. Along the longitudinal direction L, the pair 4 of stiffening elements 5, 6 are arranged between the end rings 27, 28. The stiffening elements 5, 6 are formed as stiffening bars, which extend in the longitudinal direction L, and are arranged at a distance to each other in the circumferential direction U. Sides of the stiffening elements 5, 6 of the pair pf stiffening elements 5, 6, which face each other along the circumferential direction U, are toothed.

In the exemplary embodiment of Figure 16, the default state of the stiffening device is the flexible state. Yet, Figure 16 shows the stiffening device 3 in its stiffened state S, in which neighboring stiffening elements 5, 6 contact each other such that the teeth of the two stiffening elements 5, 6 are engaged. The teeth may be designated as latch protrusions. In order to bring the stiffening elements 5, 6 of the pair 4 into engagement with each other, at least one displacement element 29, for instance a ball or a bar, may be provided, which is forced between the stiffening elements 5, 6, the displacement element 29 pushing one of the stiffening elements 5, 6 towards the other one of the stiffening elements 5, 6.

Figure 17 partly shows another exemplary embodiment of the stiffening device 3 in a perspective view. Same reference signs are being used for elements, which correspond in function and/or structure to the elements of the previous exemplary embodiments. For the sake of brevity, only the differences from the exemplary embodiment of Figure 16 are looked at.

The stiffening device 3 is formed with the end rings 27, 28 and with the bar-shaped stiffening elements 5, 6 between the end rings 27, 28. The bar-shaped stiffening elements 5, 6 may be toothed rods, whose teeth may be designated as latch protrusions that can engage each other to form a positive lock. The positive lock of teeth of the bar-shaped stiffening elements 5, 6 can be made or opened by turning the end rings 27, 28 against each other along the circumferential direction U. Alternatively, a default positive lock between the bar-shaped stiffening elements 5, 6 may be opened by introducing the displacement element 29 between the bar-shaped stiffening elements 5, 6.

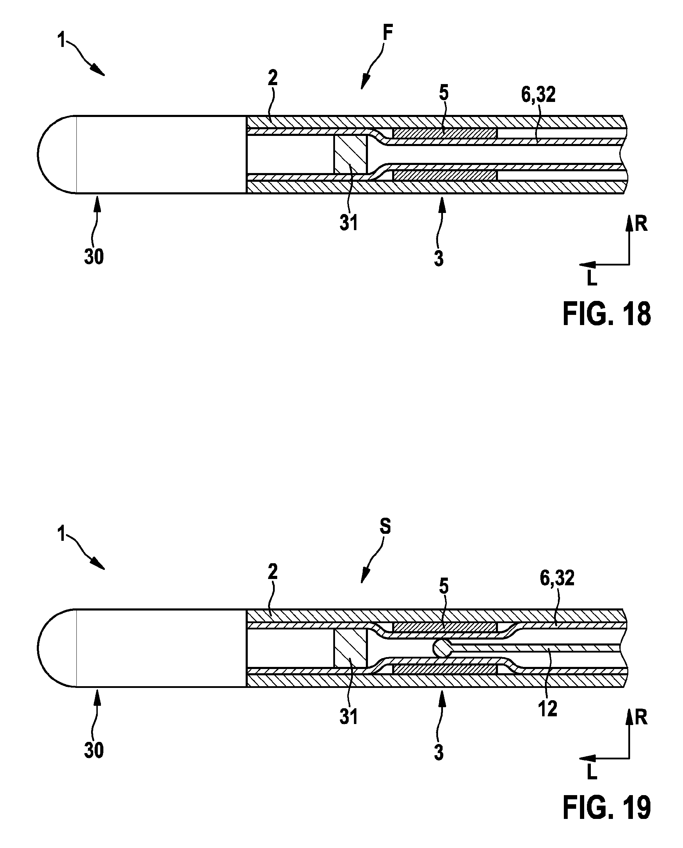

Figures 18 and 19 show a further exemplary embodiment of the device 1 in cross-sectional views. Same reference signs are being used for elements, which correspond in function and/or structure to the elements of the previous exemplary embodiments. For the sake of brevity, only the differences from the exemplary embodiments are looked at.

The device 1 of Figures 18 and 19 is exemplarily shown as an electrode with an electrode head 30 at its longitudinal end. In the longitudinal direction L before the head 30, the tube 2 is arranged, which is affixed to the head. Inside of the tube2, the stiffening device 3 according to another exemplary embodiment is arranged. Between the head 30 and the stiffening device 3, an electronic component 31 is arranged in the tube 2.

The stiffening device 3 of the exemplary embodiment of Figures 18 and 19 comprises the two stiffening elements 5, 6, wherein the stiffening element 5 is tube-shaped and arranged inside of the tube 2. The other stiffening element 6 is provided as a sleeve 32 inside of the tube-shaped stiffening element 5, the sleeve 32 being flexible in the radial direction R and less flexible or less deformable in the longitudinal direction L.

The sleeve 32 extends towards the head 30. At the longitudinal end of the device 1, the sleeve 32 preferably is affixed. Hence, in case the device 1 is bent, the sleeve 32 moves within the stiffening element 5. In the exemplary embodiment of Figure 18, the sleeve 32 can freely move in and against the longitudinal direction L, such that Figure 18 shows device 1 in its flexible state F.

In Figure 19, however, the stylet 12 is inserted in the sleeve 32 such that it presses the sleeve 32 in the radial direction against the stiffening elements 5.

Due to static friction between the sleeve 32 and the stiffening element 5, movements in the longitudinal direction L of the sleeve 32 relative to the stiffening element 5 is prevented. Hence, in case the relative movement between the sleeve 32 and the stiffening element 5 is prohibited, the stiffness of the device 1 is not only increased in the area of the stiffening element 5, but furthermore beyond the electronic component 31 as seen from the stiffening element 5. Hence, with such a device 1, the stiffness or flexibility of the device 1 can be changed in sections which cannot be reached by the stylet 12.

Alternatively, the tube 2 may form the stiffening element 5, which, hence, needs not to be provided separately.

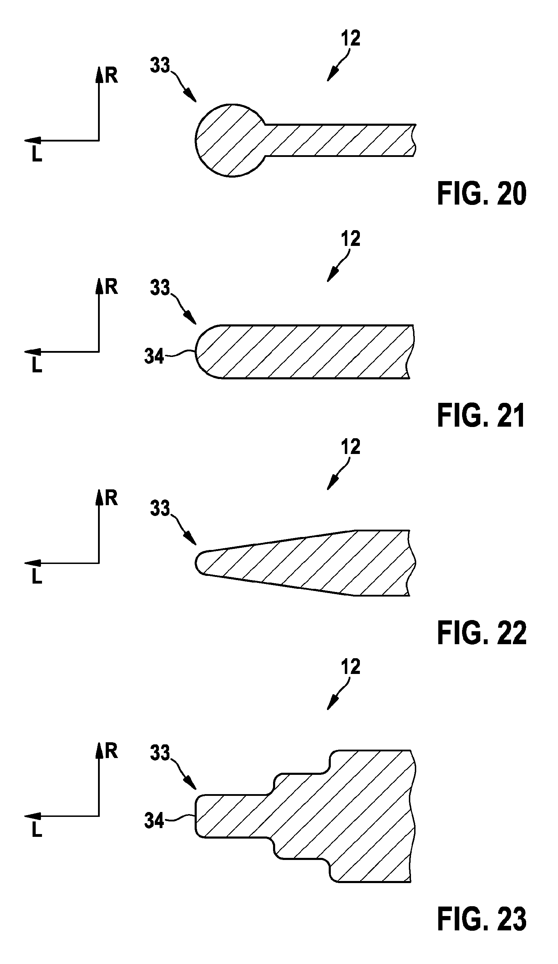

Figures 20 to 23 show exemplary embodiments of stylets 12 in cross-sectional views along the longitudinal direction L and the radial direction R. The stylets 12 may have a rotational symmetric form around the longitudinal direction L.

The stylet 12 of the exemplary embodiment of Figure 20 has a thickened end 33 which has a larger diameter in the radial direction R than a section of the stylet 12 that is arranged before the thickened end 30 in the longitudinal direction L. The thickened end 30 may have a circular cross-section and may, thus, be ball shaped.

The longitudinal end 33 of the stylet 12 shown in Figure 21 has a rounded far end 34, whose diameter, except for the rounded end 34, essentially corresponds to the diameter of the section before the longitudinal end 33 in the longitudinal direction L.

The diameter of the longitudinal end 33 of the stylet 12 shown in Figure 22 decreases continuously in the longitudinal direction L compared to a constant diameter of the section before the longitudinal end 33 in the longitudinal direction L.

According to the exemplary embodiment of Figure 23, the diameter of the longitudinal end 33 gradually decreases in the longitudinal direction. In particular, the diameter decreases in two steps from the section before the longitudinal end 33 in the longitudinal direction L to a far end 34 of the longitudinal end 33.

As an alternative to the stylet 12, a balloon may be placed inside of the tube, e.g. inside the other stiffening element 6, the balloon being inflatable at choice of the user of the device 1, for instance via a gas tube connected to the balloon in a gas transmitting manner.

## Claims

1. Device (1) for implantation or insertion into the human body, with a tube (2) through which a cavity extends in the longitudinal direction (L) of the tube, **characterized by** at least one stiffening device (3) with a pair (4) of stiffening elements (5, 6) that are adapted to be affixed to each other in a stiffened state (S) of the device (1) or to be detached from each other in a flexible state (F) of the device (1).

2. Device (1) according to claim 1, **characterized in that** the stiffening elements (5, 6) are adapted to be affixed to each other with a form fit or with a force fit.

3. Device (1) according to claim 1 or 2, **characterized in that** one of the stiffening elements (5, 6) is formed with a latch element (8), and the other one of the stiffening elements (6) is formed with a counter-latch element (9), wherein the stiffening elements (5, 6) are latched to each other in the stiffened state (S).

4. Device (1) according to claim 3, **characterized in that** in a default state, the stiffening elements (5, 6) are latched or not latched to each other.

5. Device (1) according to claim 3 or 4, **characterized in that** the latch element (8) is formed with a latch protrusion and the counter-latch element (9) is formed with a latch recess for the latch protrusion.

6. Device (1) according to any of claim 3 to 5, **characterized in that** the counter-ledge element (9) is formed with a plurality of latch recesses that are arranged after each other in the longitudinal direction (L).

7. Device (1) according to any of claims 1 to 6, **characterized in that** the device (1) comprises two pairs (4) of stiffening elements (5, 6) that are arranged opposite of each other.

8. Device (1) according to any of claims 1 to 6, **characterized in that** the device comprises more than two pairs (4) of stiffening elements (5, 6), wherein each of the pairs (4) is arranged in a corner of a polygon, the amount of corners of the polygon corresponding to the amount of pairs (4) of stiffening elements (5, 6).

9. Device (1) according to any of claims 3 to 8, **characterized in that** at least one of the latch element (8) and the counter-latch element (9) extends into the cavity.

10. Device (1) according to claim 1 or 2, **characterized in that** one of the stiffening elements (5) is formed by the tube (2) and the other one of the stiffening elements (6) is arranged in the cavity of the tube (2), wherein the stiffening elements (5, 6) are pressed against each other in the stiffening state (S).

11. Device (1) according to claim 10, **characterized in that** in the default state the stiffening elements (5, 6) are not pressed against each other.

12. Device (1) according to claim 10 or 11, **characterized in that** the other stiffening element (6) is essentially tube-shaped.

13. Device (1) according to claim 12, **characterized in that** the other stiffening element (6) is formed with a radially flexible section (6a) with at least one slit (21).

14. Device (1) according to claim 13, **characterized in that in that** the slit (21) is spirally shaped.

15. Set of implanting or inserting a device (1) into the human body, with the device (1) and a stylet (12), **characterized in that** the device (1) is the device (1) according to any of the claims 1 to 14.
